# EUROPEAN PATENT APPLICATION

(11) **EP 4 342 468 A1**
(43) Date of publication of application: **27.03.2024**
(21) Application number: 22804060.6
(22) Date of filing: 20.05.2022
(51) Int. Cl.: A61K 31/437, A61P 35/00, A61P 37/00, A61P 29/00, A61P 7/02

(54) **TOLEBRUTINIB SALT AND CRYSTAL FORM THEREOF, PREPARATION METHOD THEREFOR, PHARMACEUTICAL COMPOSITION THEREOF, AND USE THEREOF**

(30) Priority: 21.05.2021 CN 202110557261; 25.02.2022 CN 202210156938
(71) Applicant: Hangzhou Solipharma Co., Ltd., Hangzhou, Zhejiang 310018 (CN)
(72) Inventor: SHENG, Xiaohong, Hangzhou, Zhejiang 310018 (CN); SHENG, Xiaoxia, Hangzhou, Zhejiang 310018 (CN); DAI, Yu, Hangzhou, Zhejiang 310018 (CN)
(74) Representative: Müller-Boré & Partner Patentanwälte PartG mbB
(86) International application number: PCT/CN2022/094073
(87) International publication number: WO 2022/242740

(57) **Abstract**

This application relates to the chemical field of medicine, specifically to tolebrutinib salts and their crystal forms, their preparation methods, their pharmaceutical compositions, and their uses. Tolebrutinib hydrochloride and its crystal form provided in this application have at least one advantage: good stability, low hygroscopicity, solubility meeting medicinal requirements, uniform particle size distribution, good morphology, good flowability, good compressibility, good crystallinity, good storage stability, avoiding phase transformation during drug development and storage, simple and reliable preparation method, and great development value. The tolebrutinib maleate and its crystal form of this application have at least one advantage: good stability, uniform particle size distribution, good flowability, solubility meeting pharmaceutical requirements, good storage stability, avoiding phase transformation during drug development and storage, and simple and reliable preparation method.

## Description

### CROSS-REFERENCE TO RELATED APPLICATIONS

This application claims the full rights of the invention patent application with the application numbers 202110557261.3, submitted to the National Intellectual Property Administration of the People's Republic of China on May 21 2021, and 202210156938.7, submitted on February 25, 2022. The entire contents of these applications are incorporated herein by reference.

### TECHNICAL FIELD

The present application relates to the chemitry field of medicine. In particular, the present application relates to tolebrutinib salt and crystal form thereof, preparation method therefor, pharmaceutical composition thereof, and use thereof.

### BACKGROUND

Polymorph or polymorphism is a particular property of some molecules and molecular combinations. Different crystalline forms of certain compounds arise from different molecular packing in the crystal lattice, and these crystalline forms have different crystal structures and physical properties such as solubility, stability, thermal property, mechanical property, purification ability, X-ray diffraction pattern, infrared absorption spectroscopy, Raman spectroscopy, solid state nuclear magnetic resonance, etc.

The discoverying novel crystal forms of pharmaceutically active ingredients (including anhydrates, hydrates, and solvates, etc.) may lead to materials with enhanced processing advantages or improved physicochemical properties. These properties can include better bioavailability, better storage stability, better processibility, and ease of purification, or serving as intermediate forms that facilitate the transformation into other crystal forms. Some specific crystal forms of active pharmaceutical ingredients can also contribute to the improvement of drug performance. This expands the range of available material forms in formulation science, such as improving dissolution rates, extending shelf life, and facilitating easier processing.

Tolebrutinib (development code SAR442168), initially developed by Principia and later acquired by Sanofi, is a Bruton's tyrosine kinase (BTK) inhibitor. It is utilized for the treatment of various conditions, including cancer, autoimmune diseases, such as multiple sclerosis and myasthenia gravis, inflammatory diseases and thromboembolic diseases. Its structural formula is shown in (I):

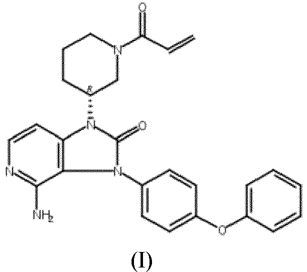

Currently, there is no publicly available information in the existing technology regarding the salt and crystal forms of this compound. Therefore, it is necessary to conduct salt and polymorph screening for Tolebrutinib, selecting salts and crystal forms with beneficial properties for the product development of Tolebrutinib.

The inventors of the present application discovered hydrochloride salt of tolebrutinib and two crystal forms of the hydrochloride salt of tolebrutinib during the researches process. The hydrochloride salt and crystal forms of Tolebrutinib provided in this application have at least one of the following advantages: excellent stability, low hygroscopicity, solubility meeting pharmaceutical requirements, uniform particle size distribution, good morphology, excellent flowability, good compressibility, good crystallinity, stable storage, prevention of polymorphic transformation during development and storage, a simple and reliable preparation method, and significant development value. The hydrochloride salt and crystal forms of Tolebrutinib provided in this application have at least one of the following advantages: excellent stability, low hygroscopicity, solubility meeting pharmaceutical requirements, uniform particle size distribution, good morphology, good flowability, good compressibility, good crystallinity, good storage stability, avoding polymorphic transformation during development and storage, a simple and reliable preparation method, and significant development value.

Additionally, the inventors of this application have discovered a maleate salt of tolebrutinib and its crystalline form during the research process. The maleate salt of tolebrutinib and the crystalline form thereof provided in the present application have at least one of the following advantages: good stability, uniform particle size distribution, good flowability, solubility meeting pharmaceutical requirements, good storage stability, avoiding polymorphic transformation during development and storage, and a simple and reliable preparation method.

### SUMMARY

The purpose of this application is to provide the hydrochloride salt and crystal forms of tolebrutinib, as well as the malate salt and its crystal forms, along with their preparation methods, pharmaceutical compositions, and uses.

One aspect of this application is to provide a tolebrutinib monohydrochloride, the structure of which is shown in formula(II):

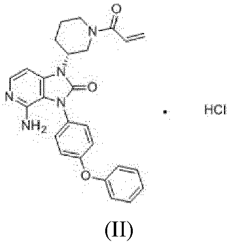

Another aspect of the present application is to provide a Form 2 of tolebrutinib monohydrochloride (hereinafter referred to as Form 2) as shown in formula (II). Using Cu-Ku radiation, the X-ray powder diffraction (XRPD) pattern of Form 2 comprises at least one characteristic peak at 20 values of 10.2° ± 0.2°, 12.1° ± 0.2°, 15.8° ± 0.2°, 16.2° ± 0.2° and 17.4° ± 0.2°; preferably at least three characteristic peaks; more preferably at least four characteristic peaks.

In a preferred embodiment of the present application, the XRPD pattern of Form 2 also comprises at least one characteristic peak at 20 values of 15.0°±0.2°, 19.2°±0.2°, 20.3°±0.2°and 21.2°±0.2°2θ.

In a preferred embodiment of the present application, the XRPD pattern of Form 2 also comprises one characteristic peak at 20 values of 8.1°±0.2°, 13.4°±0.2°, 14.5±0.2°, 23.0°±0.2°, 24.3°±0.2°and 25.1°±0.2°2θ.

In a preferred embodiment of the present application, the XRPD pattern of Form 2 comprise characteristic peaks at the following 20 values:

| 2θ±0.2° |
|---|
| 8.1 |
| 10.2 |
| 12.1 |
| 13.4 |
| 14.5 |
| 15.0 |
| 15.8 |
| 16.2 |
| 16.8 |
| 17.4 |
| 19.2 |
| 20.3 |
| 20.9 |
| 21.2 |
| 21.5 |
| 22.0 |
| 22.5 |
| 22.7 |
| 23.0 |
| 23.4 |
| 23.8 |
| 24.3 |
| 25.1 |
| 29.5 |
| 30.2 |

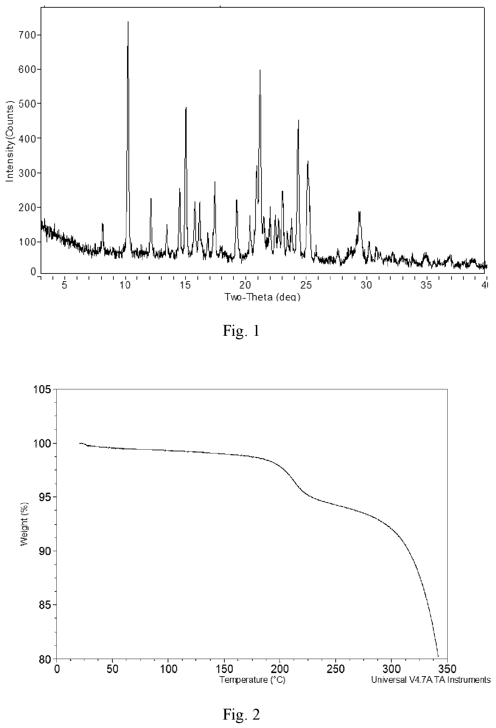

Non-restrictively, Form 2 has an XRPD pattern substantially as shown in FIG. 1.

Non-restrictively, Form 2 is anhydrous.

Non-restrictively, the Thermogravimetric Analysis (TGA) thermogram of Form 2 essentially as shown in FIG. 2, with a surface weight loss of no more than 1.0% before heating to 100°C.

In a preferred embodiment of the present application, the onset value in the DSC thermogram of Form 2 is 250°C±3°C.

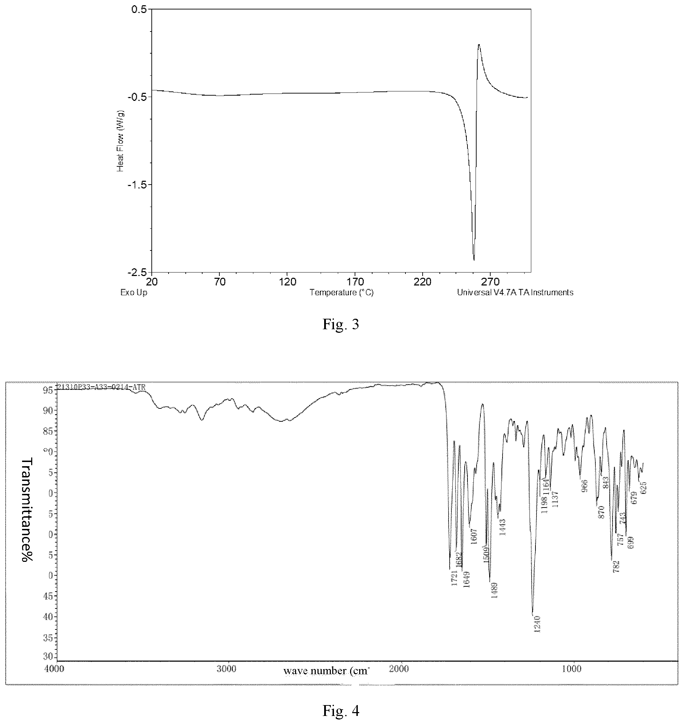

Non-restrictively, the DSC thermogram of Form 2 is substantially as shown in FIG. 3.

In a preferred embodiment of the present application, the Fourier transform infrared (FT-IR) spectrum of Form 2 has at least one characteristic peak at 1721cm⁻¹±2cm⁻¹, 1682cm⁻¹±2cm⁻¹, 1649cm⁻¹±2cm⁻¹, 1607cm⁻¹±2cm⁻¹, 1509cm⁻¹±2cm⁻¹, 1489cm⁻¹±2cm⁻¹, 1443cm⁻¹±2cm⁻¹, 1240cm⁻¹±2cm⁻¹, 870cm⁻¹±2cm⁻¹, 782cm⁻¹±2cm⁻¹, 757cm⁻¹±2cm⁻¹ and 699cm⁻¹±2cm⁻¹.

Non-restrictively, Form 2 has an FT-IR spectrum substantially as shown in FIG. 4

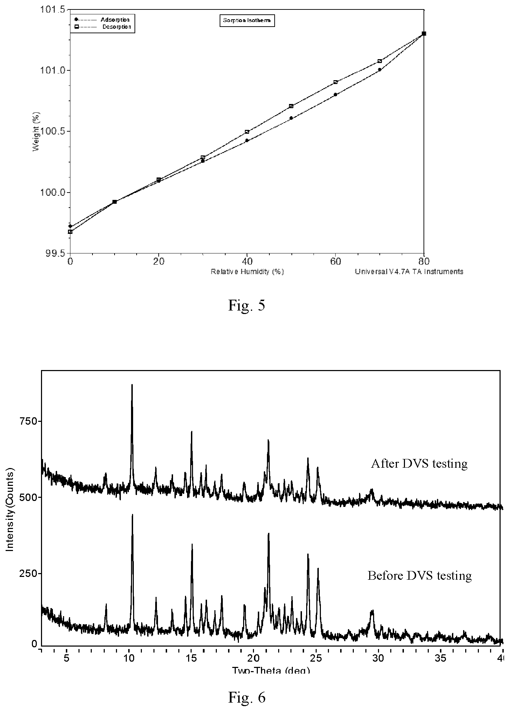

Non-restrictively, the DVS of Form 2 is substantially as shown in FIG. 5, with a weight gain about 1.0% (w/w) in the range of 30%RH-80%RH.

Another aspect of the present application is to provide a method for preparing the tolebrutinib monohydrochloride. The method comprises:Dissolving tolebrutinib in solvent 1 to form a clear solution or a supersaturated solution, then mixing it with hydrochloric acid solution to obtain the tolebrutinib monohydrochloride.

Wherein, the hydrochloric acid solution is obtained by diluting concentrated hydrochloric acid with solvent 2. Solvent 1 and solvent 2 are selected from ethanol, methanol, n-propanol, isopropanol, toluene, ethyl acetate, acetonitrile, or any combination thereof.

Preferably, the molar ratio (mol/mol) of tolebrutinib to hydrochloric acid is 1:0.8 to 1:1.2; more preferably 1:1.

Another aspect of the present application is to provide a method for preparing Form 2 of tolebrutinib monohydrochloride. The preparation method comprises:Dissolving tolebrutinib in solvent 3 to form a solution or a supersaturated solution, then mixing it with hydrochloric acid solution, stirring, separating, and drying to obtain Form 2 of tolebrutinib monohydrochloride.

Wherein, the hydrochloric acid solution is obtained by diluting concentrated hydrochloric acid with solvent 4. Solvent 3 and solv 4 are selected from ethanol, methanol, n-propanol, isopropanol, and toluene, or any combination thereof.

Preferably, both solvent 3 and solvent 4 are ethanol.

Preferably, the mass/volume ratio (mg/mL) of concentrated hydrochloric acid to solvent 4 is 100:1 to 2:1, further preferably 50:1 to 5:1, most preferably 30:1 to 10:1; the concentration of hydrochloric acid of the concentrated hydrochloric acid is 37% (w/w).

Preferably, the molar ratio of tolebrutinib to hydrochloric acid is 1:0.8 to 1:1.2.

Preferably, the stirring is performed at room temperature.

Preferably, the stirring time is equal to or greater than 2 days, more preferably it ranges from 3 to 20 days.

Tolebrutinib monohydrochloride Form 2 described in this application exhibits the following beneficial effects:
1) Tolebrutinib monohydrochloride Form 2 exhibits good stability, which is advantageous for the storage of samples and the stability of formulations. The crystal form of Form 2 of the hydrochloride salt remains unchanged under long-term and accelerated conditions for 14 days, with its chemical purity essentially maintained before and after placement. Additionally, when Form 2 of the hydrochloride salt is mixed with excipients to form a pharmaceutical formulation, the crystal form remains stable for at least 14 days under conditions of 25°C/60% RH. This observation indicates that Form 2 of the hydrochloride salt as the active ingredient and the formulations prepared using Form 2 as described in this application possess excellent storage stability.
2) Form 2 of tolebrutinib monohydrochloride exhibits low hygroscopicity, requiring no special humidity conditions during production and storage environments. It is suitable for industrial scale production, and is beneficial for the storage of the compound and its formulated products.
3) Form 2 of tolebrutinib monohydrochloride exhibits good solubility, with a solubility of approximately 7.0 mg/mL in water. This characteristic is advantageous for achieving the desired drug bioavailability and efficacy, meeting pharmaceutical requirements.
4) Form 2 of tolebrutinib monohydrochloride exhibits good compressibility, which is beneficial for formulation processes. This characteristic improves the appearance of the product and enhances overall product quality.
5) Form 2 of tolebrutinib monohydrochloride exhibits good crystallinity.
6) The preparation method of Form 2 of tolebrutinib monohydrochloride is simple, exhibits high reproducibility, and holds promising potential for industrialization.

Another aspect of the present application provides a Form 1 of tolebrutinib monohydrochloride (hereinafter referred to as Form 1) as shown in formula (II). Using Cu-Ku radiation, wherein the X-ray powder diffraction (XRPD) pattern of Form 2 comprises at least four characteristic peaks at 20 values of 6.4°±0.2°, 12.8°±0.2°, 19.0°±0.2°, 20.3°±0.2°, and 22.3°±0.2°.

In a preferred embodiment of the present application, the XRPD pattern of Form 1 also comprises one or two or more characteristic peaks at 20 values of 9.6°±0.2°, 15.0°±0.2°, 24.2°±0.2°, 25.0±0.2°and 25.6°±0.2°.

In a preferred embodiment of the present application, the XRPD pattern of Form 1 also comprises one or two or more characteristic peaks at 20 values of 11.1°±0.2°, 14.6°±0.2°, 16.5°±0.2°and19.8°±02°.

In a preferred embodiment of the present application, the XRPD pattern of Form 1 comprise characteristic peaks at the following 20 values:

| 2θ±0.2° |
|---|
| 6.4 |
| 9.6 |
| 11.1 |
| 12.4 |
| 12.8 |
| 14.6 |
| 15.0 |
| 16.5 |
| 19.0 |
| 19.8 |
| 20.3 |
| 22.3 |
| 22.7 |
| 24.2 |
| 25.0 |
| 25.3 |
| 25.6 |
| 28.1 |

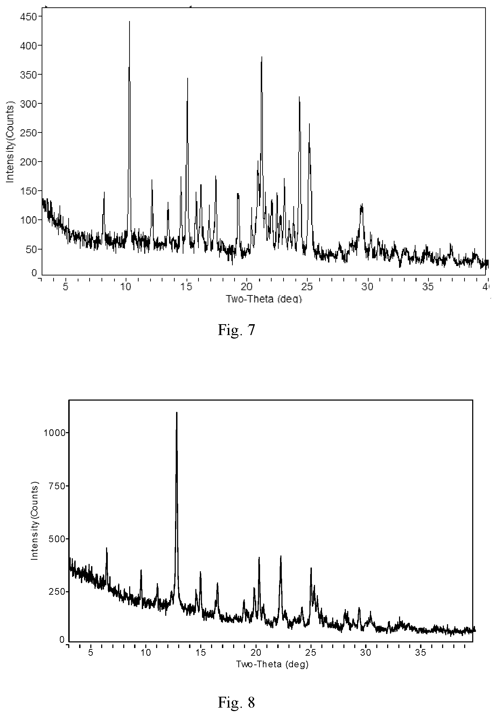

Non-restrictively, Form 1 has an XRPD pattern substantially as shown in FIG. 8.

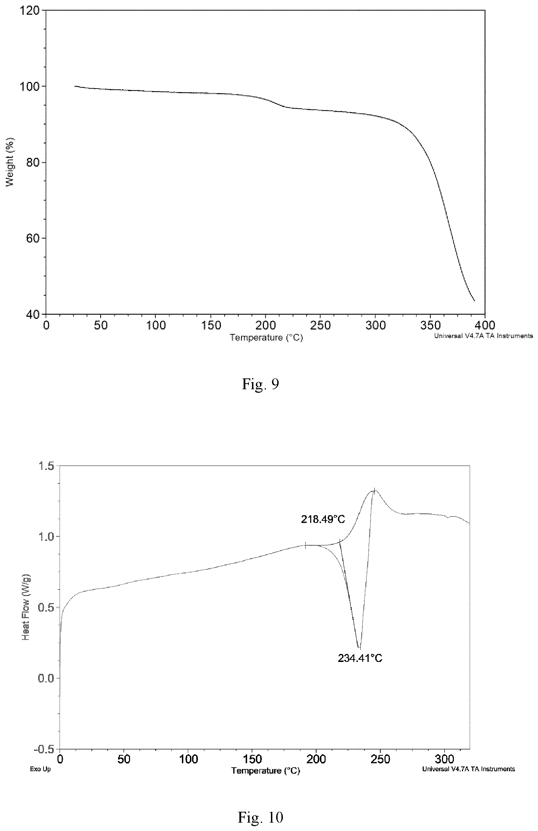

Non-restrictively,Form 1 has a TGA thermogram substantially as shown in FIG. 9.

In a preferred embodiment of the present application, the onset value in the DSC thermogram of Form 1 is 218±2°C.

Non-restrictively, Form 1 has a DSC thermogram substantially as shown in FIG. 10.

In a preferred embodiment of the present application, the Fourier transform infrared (FT-IR) spectrum of Form 1 exhibits characteristic peaks at1722±2 cm⁻¹, 1682±2 cm⁻¹, 1650±2 cm⁻¹, and 1607±2 cm⁻¹.

In a preferred embodiment of the present application, the Fourier transform infrared (FT-IR) spectrum of Form 1 also exhibits one or two or more characteristic peaks at 1509±2cm⁻¹, 1489±2 cm⁻¹, 1444±2 cm⁻¹, 1240±2 cm⁻¹, 1164±2 cm⁻¹, 1137±2 cm⁻¹, 782±2 cm⁻¹ and 699±2 cm⁻¹.

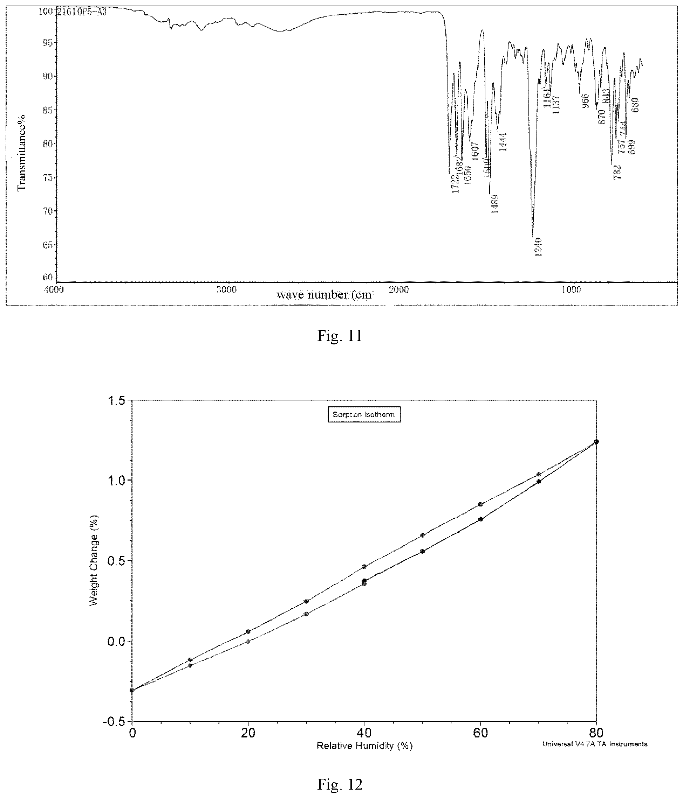

Non-restrictively, Form 1 has an FT-IR spectrum substantially as shown in FIG. 11.

Non-restrictively, Form 1 has a DVS (dynamic vapor sorption) plot substantially as shown in FIG. 12.

In a preferred embodiment of the present application, Form 1 is anhydrous.

Another aspect of the present application is to provide a method for preparing Form 1 of tolebrutinib monohydrochloride which is selected from the following methods:
1) Dissolving tolebrutinib in solvent 5 to form a solution or a supersaturated solution, then mixing it with hydrochloric acid solution, stirring, separating, and drying to obtain Form 1 of tolebrutinib monohydrochloride.

Wherein, the hydrochloric acid solution is a solution of concentrated hydrochloric acid diluted with solvent 6. Solvent 5 and solvent 6 are selected from any one of ethyl acetate, acetonitrile, and a mixture of acetonitrile and water, or a mixture a mixture of any one of ethyl acetate, acetonitrile, and water.

Preferably, both solvent 5 and solvent 6 are ethyl acetate.

Preferably, the mass/volume ratio (mg/mL) of concentrated hydrochloric acid to ethyl acetate is 100:1 to 2:1, further preferably 50:1 to 5:1, most preferably 30:1 to 10:1; the concentration of hydrochloric acid of the concentrated hydrochloric acid is 37% (w/w).

Preferably, the molar ratio of tolebrutinib to hydrochloric acid is 1:0.8-1:1.2.

Preferably, the stirring is performed at room temperature.

2) Dissolving tolebrutinib in solvent 7, then adding crystal seeds of Form 1 of tolebrutinib monohydrochloride, then mixing it with hydrochloric acid solution, stirring, separating, and drying to obtain Form 1 of tolebrutinib monohydrochloride.

Wherein, the hydrochloric acid solution is a solution of concentrated hydrochloric acid diluted with solvent 8. Solvent 7 and solvent 8 are selected from toluene, ethanol, methanol, n-propanol, isopropanol, or any combination thereof.

Preferably, the mass ratio (mg/mg) of the tolebrutinib to the seeds of Form 1 of tolebrutinib monohydrochloride is equal to or less than 10:1, further preferably in the range of 10:5 to 10:3.

Preferably, the mass/volume ratio (mg/mL) of concentrated hydrochloric acid to solvent 4 is 100:1 to 2:1, further preferably 50:1 to 5:1, most preferably 30:1 to 10:1; the concentration of hydrochloric acid of the concentrated hydrochloric acid is 37% (w/w).

Preferably, the molar ratio of tolebrutinib to hydrochloric acid is 1:0.8 to 1:1.2.

Preferably, the stirring is performed at room temperature.

Form 1 of tolebrutinib monohydrochloride in this application exhibits the following beneficial effects:
1) Form 1 of tolebrutinib monohydrochloride exhibits low hygroscopicity, with an approximate weight gain of 0.9% in the humidity range of 0%RH to 80%RH. This characteristic implies that it requires no specific humidity conditions during production and storage, making it suitable for industrial-scale production. Additionally, it is advantageous for the storage of both the compound and its formulated products.
2) Form 1 of tolebrutinib monohydrochloride exhibits good stability, which is advantageous for the storage of samples and the stability of formulations. Form 1 of the hydrochloride salt remains unchanged under long-term and accelerated conditions for 10 months, with its chemical purity essentially maintained before and after placement. Additionally, when Form 1 of the hydrochloride salt is mixed with excipients to form a pharmaceutical formulation, the crystal form remains stable for at least 14 days under conditions of 25°C/60% RH. This observation indicates that Form 1 of the hydrochloride salt as the active ingredient and the formulations prepared using Form 1 as described in this application possess excellent storage stability.
3) Form 1 of tolebrutinib monohydrochloride exhibits a favorable external appearance, presenting as a solid, powdery substance with uniform spherical particles. This characteristic is advantageous for improving flowability.
4) Form 1 of the monohydrochloride salt of tolebrutinib demonstrates good solubility, with a dissolution rate of approximately 8.3 mg/mL in water. This high solubility is advantageous for achieving the desired drug bioavailability and efficacy, meeting pharmaceutical requirements.
5) The preparation method of Form 1 of the mono hydrochloride salt of tolebrutinib is simple, exhibits high reproducibility, and holds promising potential for industrialization.

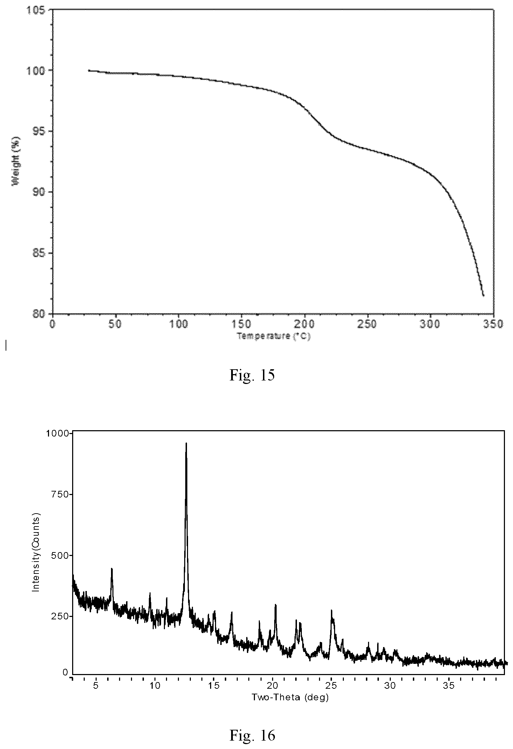

Another aspect of the present application is to provide a tolebrutinib Form 3 of monohydrochloride Form 3, which is obtained by crystallization from monohydrochloride Form 1 in water or ether, and its XRPD is as shown in FIG. 16.

Another aspect of the present application is to provide a maleate salt, wherein the maleate salt is a monomaleate salt, and the structural formula of the monomaleate salt is shown in formula (III) as follows:

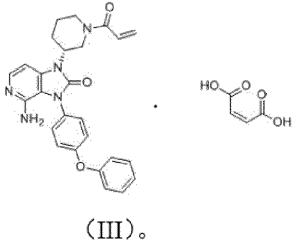

In a preferred embodiment of the present application, the tolebrutinib maleate salt is crystalline, named Form 1 of tolebrutinib maleate (hereinafter referred to as maleate Form 1). Using Cu-Ku radiation, the X-ray powder diffraction (XRPD) pattern of maleate Form 1 comprises at least four characteristic peaks at 20 values of 7.0°±0.2°, 13.4°±0.2°, 16.3°±0.2°, 17.6°±0.2°, and 20.0°±0.2°.

In preferred embodiments of the present application, the XRPD pattern of maleate Form 1 also comprises one or two or more characteristic peaks at 2θ values of 16.9±0.2°, 19.1°±0.2°, 21.9±0.2°, 25.0°±0.2°, and 25.9°±0.2°.

In a preferred embodiment of the present application, the XRPD pattern of maleate Form 1 also comprises one or two or more characteristic peaks at 20 values of 6.6°±0.2°, 10.3°±0.2°, 21.1°±0.2° and 23.0±0.2°.

In a preferred embodiment of the present application, the XRPD pattern of tolebrutinib maleate Form 1 comprise characteristic peaks at the following 20 values:

| 2θ±0.2° |
|---|
| 6.6 |
| 7.0 |
| 10.3 |
| 12.9 |
| 13.4 |
| 16.3 |
| 16.9 |
| 17.6 |
| 19.1 |
| 20.0 |
| 21.1 |
| 21.9 |
| 23.0 |
| 25.0 |
| 25.9 |

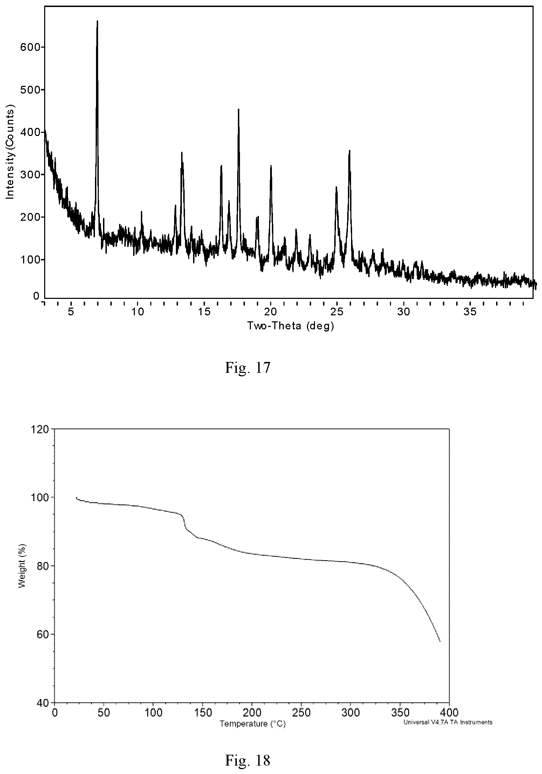

Non-restrictively,tolebrutinib maleate Form 1 has an XRPD pattern substantially as shown in FIG. 17.

Non-restrictively,tolebrutinib maleate Form 1 has a TGA thermogram substantially as shown in FIG. 18.

In a preferred embodiment of the present application, the Fourier transform infrared (FT-IR) spectrum of tolebrutinib maleate Form 1 has characteristic peaks at 1734±2cm⁻¹, 1561±2cm⁻¹, 1357±2cm⁻¹, and 1195±2cm⁻¹.

In a preferred embodiment of the present application, the Fourier transform infrared (FT-IR) spectrum of tolebrutinib maleate Form 1 also has one or two or more characteristic peaks at 1699±2cm⁻¹, 1630±2cm⁻¹, 1509±2cm⁻¹, 1486±2cm⁻¹, 1396±2cm⁻¹, 1230±2cm⁻¹, 869±2cm⁻¹ and 787±2cm⁻¹.

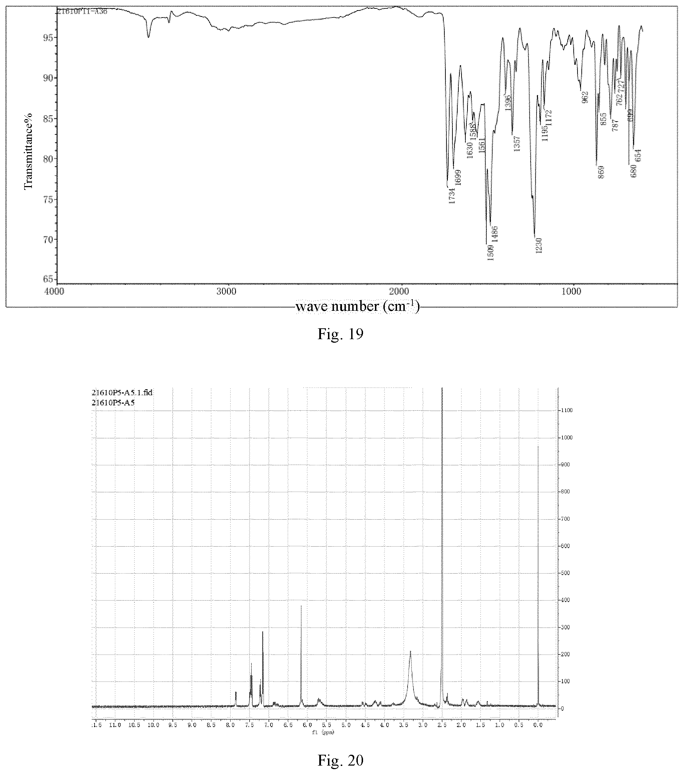

Non-restrictively, tolebrutinib maleate Form 1 has an FT-IR spectrum substantially as shown in FIG. 19.

Non-restrictively, tolebrutinib maleate Form 1 is anhydrous.

Another aspect of the present application provides a method for preparing tolebrutinib maleate salt, which comprises:
Dissolving tolebrutinib in solvent 9, then mixing it with maleic acid, stirring, separating, and drying to obtain tolebrutinib maleate Form 1.

Preferably, the molar ratio (mol/mol) of tolebrutinib to maleic acid is 1:0.8 to 1:1.2.

Preferably, solvent 9 is selected from any one of ethanol, ethyl acetate, water, tetrahydrofuran, 1,4-dioxane, ether, methanol, trifluoroethanol, n-propanol, isopropanol, n-butanol, propylene glycol, and acetone, or any combination thereof; more preferably, ethanol or ethyl acetate.

Preferably, the stirring is performed at room temperature.

Tolebrutinib maleate Form 1 provided in this aspect exhbits the following beneficial effects:
1) Tolebrutinib maleate Form 1 exhibitss good stability, which is advantageous for the storage of samples and the stability of formulations. Maleate Form 1's crystal form remains unchanged under long-term and accelerated conditions for 14 days.
2) Tolebrutinib maleate Form 1 exhibits good solubility, meeting pharmaceutical requirements.
3) Its preparation method has mild conditions, high repeatability, and holds potentials for industrialization.

Another aspect of the present application is to provide a pharmaceutical composition of tolebrutinib. The composition comprises one or more of tolebrutinib monohydrochloride salt, Form 2 of tolebrutinib monohydrochloride, Form 1 of tolebrutinib monohydrochloride, tolebrutinib maleate, tolebrutinib maleate Form 1, and at least one pharmaceutically acceptable carrier.

Another aspect of the present application is to provide a formulation prepared from the aforementioned tolebrutinib pharmaceutical composition. The formulation may exhibit various forms, including but not limited to oral solid dosage forms, topical formulations, and injectable formulations.

In a preferred embodiment of the present application, the formulation form is selected from tablets, capsules, pills, suppositories, granules, fine granules, powders sustained-release formulations, immediate-release formulations, solutions, suspensions, elixirs, and aerosols, etc.

In a preferred embodiment of the present application, the formulation form is a tablet.

The pharmaceutically acceptable carriers are commonly used excipients in the field, including but not limited to binders, adhesives, surfactants, diluents, anti-adherents, hydrophilic or hydrophobic polymers, and stabilizers, disintegrants, antioxidants, defoaming agents, fillers, glidants/lubricants, adsorbents, preservatives, plasticizers, sweeteners, and any two or more of these carriers.

In the preferred embodiment of the present application, when the formulation is an oral solid dosage form, the filler or diluent is selected from lactose, microcrystalline cellulose, starch, pregelatinized starch, calcium sulfate, calcium hydrogen phosphate, and calcium carbonate, or any combination thereof; the disintegrant is selected from sodium carboxymethyl starch, croscarmellose sodium, low-substituted hydroxypropyl cellulose, and crospovidone, or any combination thereof; the lubricant/glidant is selected from magnesium stearate, talc, and micropowder silica gel, or any combination thereof.

Furthermore, the pharmaceutical composition may also comprise one or more pH adjusting agents or buffers. For example, acids such as acetic acid, boric acid, citric acid, fumaric acid, maleic acid, tartaric acid, malic acid, lactic acid, phosphoric acid and hydrochloric acid, or any combination thereof; or bases, such as sodium hydroxide, sodium phosphate, sodium borate, sodium citrate, sodium acetate, sodium lactate, tris or any combination thereof; Or buffers such as citrate/glucose, sodium bicarbonate, ammonium chloride, or a like. Such buffers, when used as a base, may have counterions other than sodium, such as potassium, magnesium, calcium, ammonium, and other counterions; and the necessary amount of such acids, bases, and buffers to maintain the pH of the components within an acceptable range can be present in the form of a solution or solid.

Another aspect of the present application is to provide a use of one or more of tolebrutinib monohydrochloride, Form 2 of tolebrutinib monohydrochloride, Form 1 of tolebrutinib monohydrochloride, tolebrutinib maleate, tolebrutinib maleate Form 1, or the pharmaceutical composition thereof in the preparation of a drug for the treatment of BTK-mediated diseases.

Another aspect of the present application is to provide a use of one or more of Form 2 of tolebrutinib monohydrochloride, Form 1 of tolebrutinib monohydrochloride, tolebrutinib maleate Form 1, or the pharmaceutical composition thereof in the preparation of a drug for the treatment of cancers, autoimmune diseases, inflammatory diseases, and thrombotic diseases.

Another aspect of the present application is to provide a method for treating BTK-mediated diseases, comprising administering a therapeutically effective amount of one or more of Form 2 of tolebrutinib monohydrochloride, Form 1 of tolebrutinib monohydrochloride, tolebrutinib maleate Form 1, or the pharmaceutical composition thereof to a patient.

Another aspect of this application is to provide a method for treating cancers, autoimmune diseases, inflammatory diseases, and thromboembolic diseases using tolebrutinib salts and their crystal forms, and pharmaceutical composition thereof. The method comprises administering an effective amount of one or more of tolebrutinib monohydrochloride, Form 2 of tolebrutinib monohydrochloride, Form 1 of tolebrutinib monohydrochloride, tolebrutinib maleate, tolebrutinib maleate Form 1, or their pharmaceutical composition to patients in need of such treatment.

Preferably, the effective amount of one or more of tolebrutinib monohydrochloride, Form 2 of tolebrutinib monohydrochloride, Form 1 of tolebrutinib monohydrochloride, tolebrutinib maleate, tolebrutinib maleate Form 1, is in the range of 0.001 to 10 mg/kg, preferably in the range of 0.005 to 5 mg/kg.

Preferably, the method can be administered once a day, twice a day, three times a day, or more times a day; the single dose can range from 0.1 mg to 500 mg/kg/day, and the specific dose will be determined based on the actual condition of the patient.

In a preferred embodiment, the method involves once-daily administration, with a single dose of oral administration at 10, 30, 60, 90, 120, 150, 180, 210, 300, 450, or 500 mg of one or more of tolebrutinib monohydrochloride, Form 2 of tolebrutinib monohydrochloride, Form 1 of tolebrutinib monohydrochloride, tolebrutinib maleate, tolebrutinib maleate Form 1. More preferably, the dosage is 60 mg or 120 mg.

In the preferred embodiment of the present application, the diseases include but are not limited to acute necrotizing hemorrhagic leukoencephalitis, acute disseminated encephalomyelitis, Addison's disease, agammaglobulinemia, alopecia areata, alopecia universalis, amyloidosis, ankylosing spondylitis, anti-GBM/Anti-TBM nephritis, antiphospholipid syndrome (APS), antiphospholipid antibody syndrome, aplastic anemia, arthritis, autoimmune angioedema, autoimmune dysautonomia, autoimmune hepatitis, autoimmune hyperlipidemia, autoimmune immunodeficiency, autoimmune inner ear disease (AIED), autoimmune myocarditis, autoimmune oophoritis, autoimmune pancreatitis, autoimmune retinopathy, autoimmune thrombocytopenic purpura (ATP), autoimmune thyroid disease, autoimmune urticaria, autoimmune hemolytic anemia, axonal & neuronal neuropathies, Balo disease, Behcet's disease, bullous pemphigoid, cardiomyopathy, Castleman disease, celiac disease, Chagas disease, chronic fatigue syndrome, chronic inflammatory demyelinating polyneuropathy (CIDP), chronic recurrent multifocal ostomyelitis (CRMO), Churg-Strauss syndrome, cicatricial pemphigoid/benign mucosal pemphigoid, coeliac disease, Cogans syndrome, cold agglutinin disease, congenital heart block, coxsackie myocarditis, CREST disease, Crohn's disease, demyelinating neuropathies, dermatitis herpetiformis, dermatomyositis, Devic's disease (neuromyelitis optica), diabetes, discoid lupus, Dressier' s syndrome, dry eye, dysautonomia, endometriosis, eosinophilic esophagitis, eosinophilic fasciitis, erythema nodosum, essential mixed cryoglobulinemia, Evans syndrome, experimental allergic encephalomyelitis, fibromyalgia, fibrosing alveolitis, giant cell arteritis (temporal arteritis), giant cell myocarditis, glomerulonephritis, Goodpasture's syndrome, granulomatosis with polyangiitis (GPA) (formerly called Wegener's Granulomatosis), Graves' disease, Guillain-Barre syndrome, Hashimoto's thyroiditis, hemolytic anemia, Henoch-Schonlein purpura, herpes gestationis, hypogammaglobulinemia, idiopathic pulmonary fibrosis, idiopathic thrombocytopenic purpura (ITP), IgA nephropathy, IgG4-related sclerosing disease, immunoregulatory lipoproteins, inclusion body myositis, inflammatory bowel disease, interstitial cystitis, juvenile arthritis, juvenile diabetes (Type 1 diabetes), juvenile myositis, Kawasaki syndrome, Lambert-Eaton syndrome, leukocytoclastic vasculitis, lichen planus, lichen sclerosus, ligneous conjunctivitis, linear IgA disease (LAD), lupus (SLE), lupus including lupus nephritis, lyme disease, chronic, Meniere's disease, microscopic polyangiitis, mixed connective tissue disease (MCTD), mooren's ulcer, Mucha-Habermann disease, mucous membrane pemphigoid, multiple sclerosis, myasthenia gravis, myositis, narcolepsy, neuromyotonia, neutropenia, ocular cicatricial pemphigoid, opsoclonus-myoclonus syndrome, optic neuritis, Ord's thyroiditis, osteoarthritis, palindromic rheumatism, PANDAS (Pediatric Autoimmune Neuropsychiatry Disorders Associated with Streptococcus), paraneoplastic cerebellar degeneration, paroxysmal nocturnal hemoglobinuria (PNH), Parry Romberg syndrome, pars planitis (peripheral uveitis), Parsonnage-Turner syndrome, peripheral neuropathy, perivenous encephalomyelitis, pernicious anemia, pemphigus such as pemphigus vulgaris, pemphigus foliaceus, POEMS syndrome, polyarteritis nodosa, polymyalgia rheumatica, polymyositis, postmyocardial infarction syndrome, postpericardiotomy syndrome, primary sclerosing cholangitis, primary biliary cirrhosis, progesterone dermatitis, psoriasis, psoriatic arthritis, psoriaticarthritis, pure red cell aplasia, pyoderma gangrenosum, raynauds phenomenon, reactive arthritis, reflex sympathetic dystrophy, Reiter's syndrome, relapsing polychondritis, restless legs syndrome, retroperitoneal fibrosis, rheumatic fever, rheumatoid arthritis, sarcoidosis, Schmidt syndrome, scleritis, scleroderma, Sjogren's syndrome, sperm & testicular autoimmunity, stiff person syndrome, Still's disease, subacute bacterial endocarditis (SBE), Susac's syndrome, sympathetic ophthalmia, Takayasu's arteritis, temporal arteritis/Giant cell arteritis, thrombocytopenic purpura (TTP), Tolosa-Hunt syndrome, transverse myelitis, Type I, II, & III autoimmune polyglandular syndromes, ulcerative colitis, undifferentiated connective tissue disease (UCTD), uveitis, vasculitis, vesiculobullous dermatosis, vitiligo, vulvodynia, lupus, or multiple sclerosis (MS).

In the preferred embodiment of the present application, the multiple sclerosis includes but is not limited to relapsing multiple sclerosis (RMS), primary progressive multiple sclerosis (PPMS), and secondary progressive multiple sclerosis (SPMS).

One of the objects of the present application is also to provide a combination use of one or more of tolebrutinib monohydrochloride, Form 2 of tolebrutinib monohydrochloride, Form 1 of tolebrutinib monohydrochloride, tolebrutinib maleate tolebrutinib maleate Form 1, or its pharmaceutical compositions, with other drugs.

The other drugs include, but are not limited to, any one or more of anticancer drugs, corticosteroids, non-corticosteroids, immunosuppressants, anti-inflammatory drugs, and combinations thereof.

Preferably, the other drugs is selected from ibrutinib, acalatinib, zanubrutinib, Velexbru and orelabrutinib.

Unless otherwise specified:
The experimental operating temperature generally refers to room temperature, and "room temperature" refers to a temperature between 10°C and 30°C.

"Stirring" may be carried out by conventional methods in the art. For example, stirring includes magnetic stirring, mechanical stirring, and the like. and the stirring speed is 50-1800 rpm, preferably 300-900 rpm.

"Separation" may be carried out by conventional methods in the art, such as centrifugation or filtration. "Separation" may employ conventional methods in the art, such as centrifugation or filtration. Preferred vacuum filtering, generally at a pressure less than atmospheric pressure for filtration, preferably less than 0.09MPa.

"Drying" can be carried out by conventional techniques in the art, such as normal temperature drying, blast drying or reduced pressure drying. Reduced pressure or atmospheric pressure may be used. Reduced pressure, preferably with a pressure less than 0.09MPa. The drying apparatus and method are not limited, and may be a fume hood, a blast oven, a spray dryer, a fluidized bed drying, or a vacuum oven, which may also be carried out under reduced or non-reduced pressure, preferably with a pressure less than 0.09MPa.

The term "concentrated hydrochloric acid" refers to various hydrochloric acid solutions commercially available, generally referring to a 37% (w/w) hydrochloric acid aqueous solution, and also includes hydrochloric acid acetone solution, hydrochloric acid ethanol solution, hydrochloric acid isopropanol solution, and hydrochloric acid 1,4-dioxane solution.

Unless otherwise specified, the proportions involved in this application, between liquid and solid, are mass/volume ratios, and proportions between liquids are volume ratios.

### DESCRIPTION OF THE DRAWINGS

FIG. 1 is an XRPD pattern of the sample of Example 2-1 (Form 2 of tolebrutinib monohydrochloride);
FIG. 2 is a TGA thermogram of the sample of Example 2-1;
FIG. 3 is a DSC thermogram of the sample of Example 2-1;
FIG. 4 is an FT-IR spectrum of the sample of Example 2-1;
FIG. 5 is a DVS plot of the sample of Example 2-1;
FIG. 6 is a comparison of XRPD before and after DVS characterization of samples in Examples 2-1;
FIG. 7 is an XRPD pattern of the sample of Example 2-2;
FIG. 8 is an XRPD pattern of the sample of Example 3-1 (Form 1 of tolebrutinib monohydrochloride);
FIG. 9 is a TGA thermogram of the sample of Example 3-1;
FIG. 10 is a DSC thermogram of the sample of Example 3-1;
FIG. 11 is an FT-IR spectrum of the sample of Example 3-1;
FIG. 12 is a DVS plot of the sample of Example 3-1;
FIG. 13 is a PLM image of the sample of Example 3-1;
FIG. 14 is an XRPD pattern of the sample of Example 3-2;
FIG. 15 is a TGA thermogram of the sample of Example 3-2;
FIG. 16 is an XRPD pattern of the sample of Example 4-2 (tolebrutinib hydrochloride Form 3);
FIG. 17 is an XRPD pattern of the sample of Example 5-1 (tolebrutinib maleate Form 1);
FIG. 18 is a TGA thermogram of the sample of Example 5-1;
FIG. 19 is an FT-IR spectrum of the sample of Example 5-1;
FIG. 20 is an 1H-NMR spectrum of the sample of Example 5-1;
FIG. 21 shows an XRPD pattern overlay of the sample (Form q of tolebrutinib maleate) of Example 5-1 before and after storaging for 14 days under long-term (25°C/60%RH/open) and accelerated conditions;
FIG. 22 shows an XRPD pattern overlay of the sample (Form 2 of tolebrutinib monohydrochloride) of Example 2-1 before and after storaging for 14 days under long-term (25°C/60%RH/open) and accelerated conditions;
FIG. 23 shows an XRPD pattern overlay of the sample (Form 1 of tolebrutinib monohydrochloride) of Example 3-1 before and after storaging for 10 months under long-term and accelerated conditions;
FIG. 24 shows an XRPD pattern overlay of the sample (Form 2 of tolebrutinib monohydrochloride) of Example 2-1 before and after the formulation;
FIG. 25 shows an XRPD pattern overlay of the sample (Form 1 of tolebrutinib monohydrochloride) of Example 3-1 before and after the formulation;
FIG. 26 shows an XRPD pattern overlay of the tablet stability sample (Form 2 of tolebrutinib monohydrochloride) of Example 2-1; and
FIG. 27 shows an XRPD pattern overlay of the tablet stability sample (Form 1 of tolebrutinib monohydrochloride) of Example 2-1.

### DETAILED DESCRIPTION

The technical implementations of the present application are described in detail below with reference to the accompanying drawings and embodiments. However, this does not limit the scope of this application to the specific embodiments described.

In this application, X-ray powder diffraction (XRPD) data were collected using a BrukerD8 Advance diffractometer; parameters are as follows: Cu radiation; wavelength: 1.54Å; Current voltage: 40KV, 40mA; Scan range: 3 to 40°2θ.

In this application, the thermogravimetric analysis (TGA) data were collected using a TA Instruments Q500 TGA; the parameters were as follows: high resolution mode; heating rate: 10°C/min; gas: N2; sample pan: platinum crucible.

In this application, Differential Thermal Analysis (DSC) data were obtained using a TA Instruments Q200 DSC; parameters were as follows: ramp rate: 10°C/min; gas: N₂; sample pan: aluminum pan with lid.

In this application, dynamic moisture adsorption analysis (DVS) data and isothermal adsorption analysis data were obtained using a TA Instruments Q5000 TGA; parameters are as follows: temperature: 25°C; relative humidity range: 0%RH-80%RH; dm/dt =0.001%/min; equilibration time: 90min; gas: N2; sample pan: platinum pan.

In this application, Fourier transform infrared spectroscopy (FT-IR) data were collected using a Bruker Tensor 27; the parameters were as follows: ATR method, collection range 600cm-1-4000cm-1, resolution 4cm-1.

In this application, the polarized light microscope (PLM) images were collected using a XPV-990E polarized light microscope; a small amount of powder sample was placed on a glass slide, a small amount of mineral oil was added dropwise to disperse the sample, a cover glass was placed on the slide, and the sample was placed on the slide. Observing and taking pictures on the stage.

In this application, proton nuclear magnetic resonance ( 1H NMR) data were obtained from a Bruker Ascend 500M HZ nuclear magnetic resonance spectrometer. A suitable amount of sample was weighed, dissolved in about 0.5 mL of deuterated dimethyl sulfoxide reagent in a nuclear magnetic sample tube.

In this application, the HPLC parameters for chemical stability and solubility are as follows:

| High Performance Liquid Chromatography (HPLC): | | | |
|---|---|---|---|
| Column | Titank-C18 3µm (100*4.6 mm) | | |
| Column temperature: | 40°C | | |
| Flow rate | 1.0 mL/min | | |
| Wavelength | 254 nm | | |
| Mobile phase | Mobile phase A: 0.01% TFA in H2O | | |
| Diluent | ACN : H2O=1:1 (v/v) | | |
| Gradient | time (min) | A (%) | B (%) |
| | 0 | 95 | 5 |
| | 0.5 | 95 | 5 |
| | 8.0 | 5 | 95 |
| | 8.1 | 95 | 5 |
| | 12 | 95 | 5 |

In the following embodiments, experimental methods without specific conditions are conducted using standard procedures and conditions, or as instructed in commercial manuals. Unless otherwise specified, the reagents and materials used in this application are commercially available.

In the present application, the starting material tolebrutinib can be obtained from the market or prepared according to prior art, such as the preparation method disclosed in WO2016196840A1.

### Example 1-1: Preparation of tolebrutinib monohydrochloride

Approximately 500mg of tolebrutinib sample was taken and dispersed in 2 mL of ethanol. 106.66mg of concentrated hydrochloric acid was weighed and diluted with 2 mL of ethanol. The hydrochloric acid solution was slowly added to tolebrutinib, resulted a solid. Confirmed by ion chromatography to obtain tolebrutinib hydrochloride.

### Example 2-1: Preparation of Form 2 of tolebrutinib monohydrochloride

Weighed approximately 50 mg of tolebrutinib sample and added 0.5 mL of ethanol to it to form a solution. Weighted 10.67 mg of concentrated hydrochloric acid and diluted it with 0.5 mL of ethanol to form a hydrochloric acid solution. The hydrochloric acid solution was slowly added to the tolebrutinib solution and stirred at room temperature for 3 days before centrifugation. After centrifugation, the sample was vacuum dried at room temperature for about 16 hours to obtain Form 2 of tolebrutinib monohydrochloride.

Its XRPD data is shown in the table below:

| 2θ | I% |
|---|---|
| 8.1 | 13.3 |
| 10.2 | 100.0 |
| 12.1 | 24.2 |
| 13.4 | 13.8 |
| 14.5 | 28.9 |
| 15.0 | 63.5 |
| 15.8 | 23.0 |
| 16.2 | 23.5 |
| 16.8 | 9.4 |
| 17.4 | 31.4 |
| 19.2 | 25.8 |
| 20.3 | 16.7 |
| 20.9 | 36.5 |
| 21.2 | 77.9 |
| 21.5 | 15.4 |
| 22.0 | 18.0 |
| 22.5 | 16.0 |
| 22.7 | 14.8 |
| 23.0 | 27.0 |
| 23.4 | 100 |
| 23.8 | 16.0 |
| 24.3 | 57.5 |
| 25.1 | 41.3 |
| 29.5 | 20.2 |
| 30.2 | 8.5 |

Its XRPD pattern is shown in FIG. 1, and it was confirmed as tolebrutinib monohydrochloride by ion chromatography.

Its TGA thermogram is shown in FIG. 2.

Its DSC thermogram is shown in FIG. 3.

Its FT-IR spectrum is shown in FIG. 4.

Its DVS curve is shown in FIG. 5, showing a weight gain of approximately 1.0% in humidity ranging from 30% RH to 80% RH. The crystal form before and after DVS remained unchanged, both of which were Form 2. The XRPD comparison plot before and after testing is shown in FIG. 6.

Form 2 of tolebrutinib monohydrochloride in this application is anhydrous.

### Example 2-2: Preparation of Form 2 of tolebrutinib monohydrochloride

Weighted approximately 20 mg of tolebrutinib sample, added 0.5 mL of ethanol to it to form a solution. Weighted 7.72 mg of concentrated hydrochloric acid and diluted it with 0.5 mL of ethanol. The diluted liquid acid was slowly added to the tolebrutinib solution, stirred the solution at room temperature for 2 days, and centrifuged. After centrifugation, the sample was vacuum dried at room temperature for about 16 hours to obtain Form 2 of tolebrutinib monohydrochloride.

Its XRPD data is shown in the table below:

| 2θ | I% |
|---|---|
| 8.2 | 23.7 |
| 10.3 | 100.0 |
| 12.2 | 30.3 |
| 13.5 | 20.0 |
| 14.6 | 31.1 |
| 15.1 | 76.3 |
| 15.8 | 25.0 |
| 16.2 | 28.4 |
| 16.9 | 20.3 |
| 17.5 | 34.7 |
| 19.3 | 27.4 |
| 20.4 | 17.6 |
| 20.9 | 38.9 |
| 21.2 | 87.4 |
| 21.5 | 24.2 |
| 22.0 | 20.0 |
| 22.5 | 24.7 |
| 22.7 | 14.2 |
| 23.1 | 31.6 |
| 23.5 | 12.6 |
| 23.8 | 19.2 |
| 24.3 | 69.5 |
| 25.2 | 58.7 |
| 29.6 | 21.3 |
| 30.2 | 11.1 |

Its XRPD pattern is shown in FIG.7.

### Example 2-3: Preparation of Form 2 of tolebrutinib monohydrochloride

The ethanol in Examples 2-1 was replaced with methanol, n-propanol, isopropanol, or toluene, while other conditions remained unchanged, resulted Form 2 of tolebrutinib monohydrochloride.

### Example 2-4: Preparation of Form 2 of tolebrutinib monohydrochloride

The room temperature stirring time in Example 2 was replaced with 5, 10, 15, or 20 days, while other conditions remained unchanged, resulted in Form 2 of tolebrutinib monohydrochloride.

### Example 3-1: Preparation of Form 1 of tolebrutinib monohydrochloride

Weighted approximately 30 mg of tolebrutinib, added 0.5 mL of ethyl acetate to it to form a solution. Added 0.5 mL of ethyl acetate to 7.7 mg of concentrated hydrochloric acid. The diluted hydrochloric acid was slowly added to the tolebrutinib solution, stirred overnight at room temperature, transferred to 5 °C for 2 days, centrifuged to precipitate the solid, and vacuum dried the solids overnight at room temperature to obtain the Form 1 of tolebrutinib monohydrochloride.

Its XRPD data is shown in the table below:

| 2θ | I% |
|---|---|
| 6.4 | 20.1 |
| 9.6 | 16.6 |
| 11.1 | 10.4 |
| 12.4 | 7.2 |
| 12.8 | 100.0 |
| 14.6 | 9.9 |
| 15.0 | 20.8 |
| 16.5 | 17.2 |
| 19.0 | 10.2 |
| 19.8 | 16.6 |
| 20.3 | 33.3 |
| 22.3 | 34.8 |
| 22.7 | 7.2 |
| 24.2 | 9.4 |
| 25.0 | 28.2 |
| 25.3 | 19.6 |
| 25.6 | 14.9 |
| 28.1 | 8.8 |

Its XRPD pattern is shown in FIG. 8. Form 1 of hydrochloride was confirmed as monohydrochloride by ion chromatography.

Its TGA thermogram was shown in FIG. 9.

Its DSC thermogram was shown in FIG. 10.

Its FT-IR spectrum was shown in FIG. 11.

Its DVS plot is shown in FIG. 12, showing a weight change of about 1.2% between 20% RH to 80% RH at 25 °C .

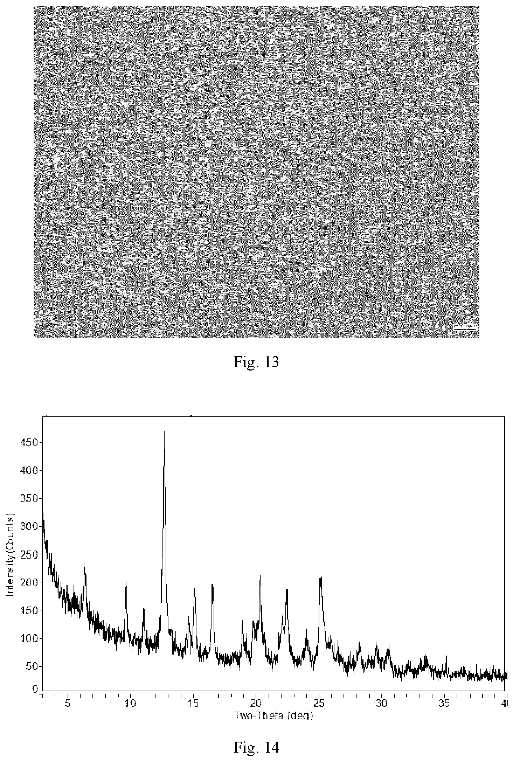

Its PLM image is shown in FIG. 13, showing small particles and uniform size distribution in the sample.

Form 1 of tolebrutinib monohydrochloride in this application is anhydrous.

### Example 3-2: Preparation of Form 1 of tolebrutinib monohydrochloride

Weighted approximately 30mg of tolebrutinib, added 0.5 mL of toluene to form a solution, about 3mg of Form 1 of tolebrutinib hydrochlorid prepared in Example 3-1 was added as seeds. 7.6mg of concentrated hydrochloric acid was diluted with 0.5 mL of toluene, and the diluted hydrochloric acid was slowly added to the tolebrutinib solution. The solution was stirred overnight at room temperature, centrifuged, and solids were vacuum dried overnight at room temperature to obtain Form 1 of tolebrutinib hydrochloride.

Its XRPD data is shown in the table below:

| 2θ | I% |
|---|---|
| 6.4 | 21.7 |
| 9.7 | 27.9 |
| 11.1 | 17.6 |
| 12.7 | 100.0 |
| 14.6 | 18.3 |
| 15.1 | 33.1 |
| 16.5 | 36.2 |
| 18.9 | 19.4 |
| 19.8 | 15.5 |
| 20.3 | 38.8 |
| 22.1 | 22.7 |
| 22.5 | 36.7 |
| 24.0 | 15.5 |
| 25.2 | 38.5 |
| 28.3 | 10.6 |

Its XRPD pattern is shown in FIG. 14.

Its TGA thermogram is shown in FIG. 15

### Example 3-3: Preparation of Form 1 of tolebrutinib monohydrochloride

The ethyl acetate in Examples 3-1 was replaced with acetonitrile or acetonitrile/water (9:1), while other conditions remained unchanged, resulted Form 1 of tolebrutinib monohydrochloride .

### Example 3-4: Preparation of Form 1 of tolebrutinib monohydrochloride

The toluene in examples 3-2 was replaced with ethanol, methanol, n-propanol, or isopropanol, while other conditions remained unchanged, resulted in Form 1 of tolebrutinib monohydrochloride .

### Example 4-1: Preparation of Form 3 of tolebrutinib monohydrochloride

About 20 mg of Form 1 of tolebrutinib hydrochloride sample prepared from Example 3-1 was added to 0.5 mL of ether room temperature, stirred overnight, centrifuged, and vacuum dried overnight at room temperature to obtain Form 3 of tolebrutinib monohydrochloride.

Its XRPD pattern is shown in Figure 16.

### Example 5-1: Preparation of Form 1 of tolebrutinib maleate

About 30 mg of tolebrutinib was dissolved in 0.5 mL of ethanol, and the tolebrutinib solution was added to a small bottle containing about 9.1 mg of maleic acid. After stirring overnight at room temperature, the solid separated by centrifugation was dried overnight under vacuum at room temperature to obtain the tolebrutinib maleate Form 1.

Its XRPD data is shown in the table below:

| 2θ° | I% |
|---|---|
| 6.6 | 10.2 |
| 7.0 | 100.0 |
| 10.3 | 14.1 |
| 12.9 | 20.0 |
| 13.4 | 40.4 |
| 16.3 | 40.2 |
| 16.9 | 24.5 |
| 17.6 | 67.3 |
| 19.1 | 20.6 |
| 20.0 | 43.3 |
| 21.1 | 10.2 |
| 21.9 | 15.7 |
| 23.0 | 15.5 |
| 25.0 | 35.7 |
| 25.9 | 51.2 |

Its XRPD pattern is shown in FIG. 17.

Its TGA thermogram is shown in FIG. 18.

Its FT-IR spectrum s shown in FIG. 19.

Its 1H-NMR spectrum is shown in FIG. confirming being tolebrutinib monomaleate

The tolebrutinib maleate Form 1 in this application is anhydrous.

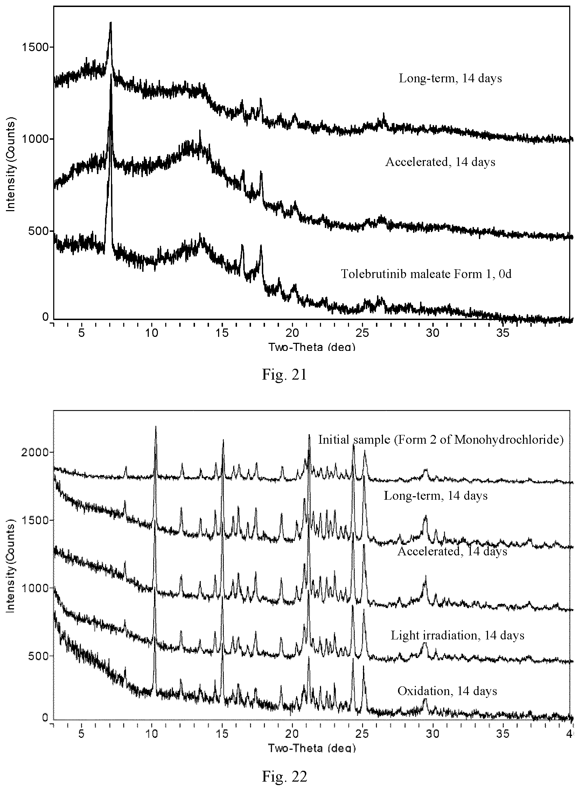

The sample was subjected to a stability test (20 mg of sample was taken in a flat dish and placed under long-term (25°C/60%RH) and accelerated (40°C/75%RH) conditions. The results showed that maleate Form 1 remained the same crystalline form for 14 days under long-term and accelerated conditions, as shown in FIG. 21.

### Example 5-2: Preparation of tolebrutinib maleate Form 1

50mg of tolebrutinib was dissolved in 0.5mL of ethanol, and the tolebrutinib solution was added to a small bottle containing approximately 15.2mg of maleic acid. After stirring at room temperature overnight, centrifugation was carried out to precipitate the solid. After vacuum drying at room temperature overnight, a sample of tolebrutinib maleate salt Form 1 was obtained.

### Example 5-3: Preparation of tolebrutinib maleate Form 1

About 20mg of tolebrutinib was dissolved in 0.5mL of ethyl acetate. The tolebrutinib solution was added to a small bottle containing about 9.1 mg of maleic acid. After stirring overnight at room temperature, solid precipitates were obtained. After centrifugation and vacuum drying overnight at room temperature, the tolebrutinib maleate Form 1 sample was obtained.

### Example 6: Stability Study of Form 2 of tolebrutinib monohydrochloride and Form 1 of tolebrutinib monohydrochloride

An appropriate amount of Form 2 of tolebrutinib monohydrochloride prepared in example 2-1 of this application was placed under long-term (25°C/60% RH/open), accelerated (40°C/75% RH/open), light (25°C/4500lx/closed), and oxidation (in a urea peroxide atmosphere) conditions. HPLC and XRPD were regularly tested, and the results are shown in Table 1 and FIG. 22.

**Table 1 Stability of Form 2 of tolebrutinib monohydrochloride**

| Initial sample | Initial purity | Placing conditions | Placing time | Sample after placing | Purity after |
|---|---|---|---|---|---|
| Form 2 of monohydrochloride | 99.50% | 25°C/60%RH/open | 14 days | Form 2 of monohydrochloride | 99.26% |
| Form 2 of monohydrochloride | 99.50% | 40°C/75%RH/ open | 14 days | Form 2 of monohydrochloride | 99.30% |
| Form 2 of monohydrochloride | 99.50% | 25°C/45001x/closed | 14 days | Form 2 of monohydrochloride | 99.35% |
| Form 2 of monohydrochloride | 99.50% | In a urea peroxide atmosphere | 14 days | Form 2 of monohydrochloride | 99.07% |

The results showed Form 2 of monohydrochloride remains stable for at least 14 days under long-term (25°C/60% RH/open) and accelerated (40°C/75% RH/open) conditions, and its chemical purity remains basically unchanged before and after placement; In addition, Form 2 of monohydrochloride is also stable for at least 14 days under light irradiation (25°C/4500lx/closed) and oxidation (in a urea peroxide atmosphere) conditions, and the chemical purity of Form 2 remains basically unchanged before and after placement.

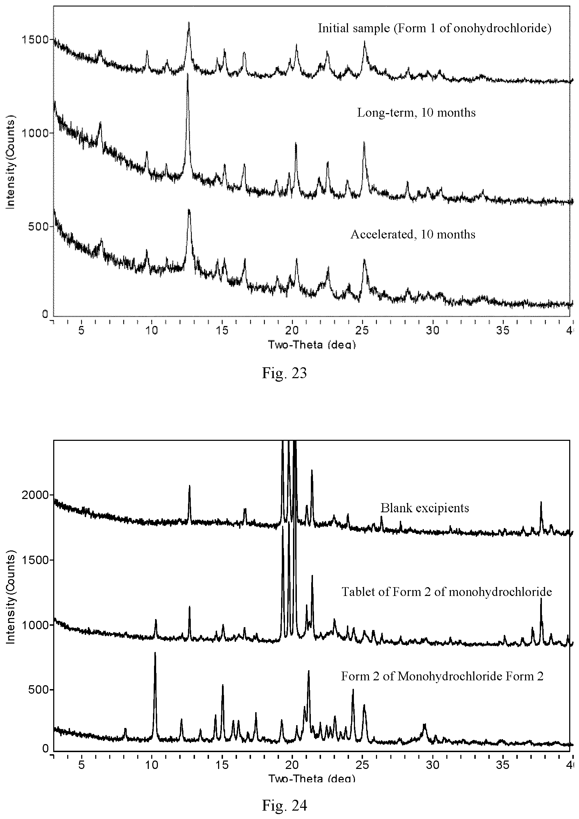

An appropriate amount of tolebrutinib monohydrochloride Form1 sample prepared in Example 3-1 of this application was taken and placed under long-term (25°C/60% RH/open) and accelerated (40°C/75% RH/open) conditions, HPLC and XRPD testing were regularly conducted. The results are shown in Table 2 and FIG. 23.

**Table 2 Stability of Form 1 of tolebrutinib monohydrochloride**

| Initial sample | Initial purity | Placing conditions | Placing time | sample after placing | Purity after placing |
|---|---|---|---|---|---|
| Form 1 of monohydrochloride | 99.46% | 25°C/60%RH/ open | 10 months | Form 1 of monohydrochloride | 99.37% |
| Form 1 of monohydrochloride | 99.46% | 40°C/75%RH/ open | 10 months | Form 1 of monohydrochloride | 98.63% |

The results showed that Form 1 of tolebrutinib monohydrochloride remains stable for at least 10 months under long-term (25°C/60% RH/open) and accelerated (40°C/75% RH/open) conditions, and the chemical purity of Form 1 of monohydrochloride remains basically unchanged before and after placement; In addition, the sample of monohydrochloride Form 1 is stable for at least 14 days under conditions of illumination (25°C/4500lx/closed) and oxidation (in a urea peroxide atmosphere), respectively, and the chemical purity of Form 1 of monohydrochloride remains basically unchanged before and after placement.

### Example 7: Solubility Study of Form 2 of monohydrochloride and Form 1 of monohydrochloride

Prepared supersaturated solutions of Form 2 of monohydrochloride and Form 1 of monohydrochloride using water, respectively, and its content in the saturated solution was determined by high-performance liquid chromatography (HPLC) at a fixed time point. The results are shown in Table 4:

**Table 4 Solubility of Form 2 of monohydrochloride and Form 1 of tolebrutinib monohydrochloride in water**

| | Sample | Time | Water |
|---|---|---|---|
| Solubility (mg/mL) | Form 2 of tolebrutinib monohydrochloride | 2 hours | 6.99 |
| | Form 1 of tolebrutinib monohydrochloride | 2 hours | 8.26 |

The results indicated that the solubility of Form 2 of tolebrutinib monohydrochloride and Form 1 of tolebrutinib monohydrochloride in water meets the pharmaceutical requirements This characteristic is advantageous for achieving the desired drug bioavailability and efficacy.

### Example 8: A Study on the Compressibility of Form 2 of tolebrutinib monohydrochloride and Form 1 of tolebrutinib monohydrochloride Form 1

The tablet compression was conducted using a Tianxiang rotary tablet press. A circular punch with a diameter of 7.5 mm (Φ7.5mm), a filling depth of 17.9 mm and a mean main pressure of 1.8 KN were used. The tablets' radial breaking force (hardness, H) was measured using a tablet hardness tester. The tablets' diameter (D) and thickness (L) were measured using a vernier caliper. The tensile strength (T) of the powder was calculated using the formula T=2H/πDL*9.8. The results are shown in Table 5:

**Table 5 Compressibility of Form 2 of tolebrutinib monohydrochloride and Form 1 of tolebrutinib monohydrochloride**

| | Form 2 of monohydrochloride | Form 1 of monohydrochloride |
|---|---|---|
| diameters (mm) | 7.50 | 7.49 |
| thicknesses (mm) | 4.23 | 4.19 |
| hardness (kg) | 5.33 | 6.16 |
| tensile strength (MPa) | 1.0 | 1.2 |

The results indicated thatthe compressibility of Form 2 of tolebrutinib monohydrochloride and Form 1 of tolebrutinib monohydrochloride Form 1 both have superior compressibility and met the pharmaceutical requirements.

### Example 7: Preparation of tablets

Following the formulation in Table 6, API, lactose hydrate, polyvinylpyrrolidone, hydroxypropyl cellulose, microcrystalline cellulose and magnesium stearate were mixed and compressed to obtain a tablet core. Subsequently, the tablet cores were coated with a solution/suspension for film coating.

**Table 6 Tablet formulations**

| | Component | Per tablet (mg) | Per tablet (mg) |
|---|---|---|---|
| Tablet Core | API | 65 | 130 |
| | Microcrystalline cellulose | 23.5 | 42 |
| | Lactose hydrate | 90 | 185 |
| | Polyvinylpyrrolidone K30 | 12.5 | 22 |
| | Hydroxypropyl cellulose | 12.5 | 28 |
| | Sodium starch glycolate | 1.5 | 3 |
| | Total mixed sample (Tablet Core Weight) | 205 | 410 |
| Film Coating | Hydroxypropyl methylcellulose | 3.5 | 7.0 |
| | Polyethylene glycol 6000 | 0.8 | 1.6 |
| | Talc | 1.3 | 2.6 |
| | Iron oxide (yellow) | 0.8 | 1.6 |
| | Titanium dioxide | 0.8 | 1.6 |
| Note: The APIs were Form 2 of monohydrochloride and Form 1 of monohydrochloride, respectively. 65mg Form 1 or Form 2 of monohydrochloride is equivalent to 60mg of the free base compound, and 130mg Form 1 or Form 2 of monohydrochloride is equivalent to 120mg of the free base compound. | | | |

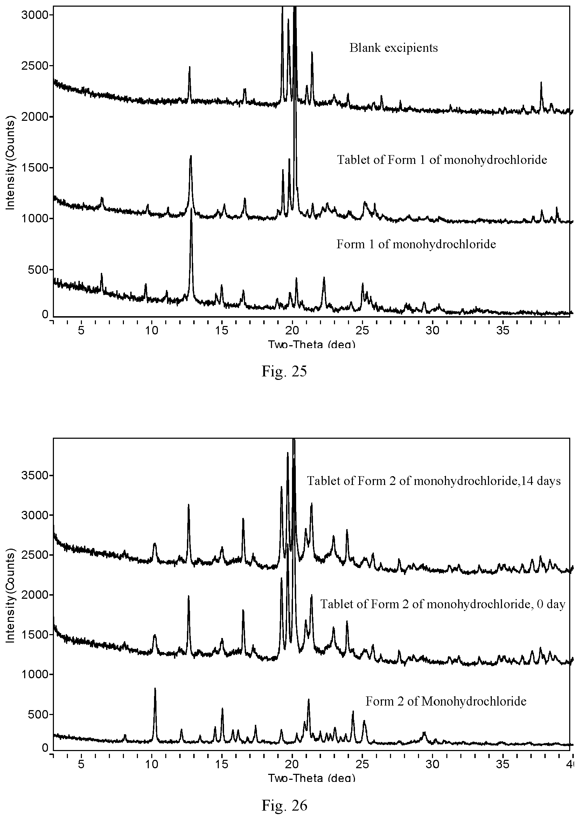

The crystal forms of the Form 2 of tolebrutinib hydrochloride sample and Form 1 of tolebrutinib hydrochloride remained unchanged before and after the tableting process, and the XRPD comparison plots were shown in FIG. 24 and FIG. 25, respectively.

### Example 10: Preparation of capsules

The capsules were prepared by sieving and mixing the components in hard gelatin capsules according to the formulation in Table 7.

**Table 7 Capsule formulation**

| Component | Per capsule (mg) | Per capsule (mg) |
|---|---|---|
| API | 65 | 130 |
| Lactose | 50 | 100 |
| Sodium starch glycolate | 0.8 | 1.5 |
| Note: The APIs were Form 2 of monohydrochloride and Form 1 of monohydrochloride respectively. 65mg Form 2 or Form 1 monohydrochloride is equivalent to 60mg of the free base compound and 130mg Form 2 of Form 1 monohvdrochloride is equivalent to 120mg of the free base compound. | | |

### Example 11: Stability of Form 2 of tolebrutinib monohydrochloride and Form 1 of tolebrutinib monohydrochloride in Tablets

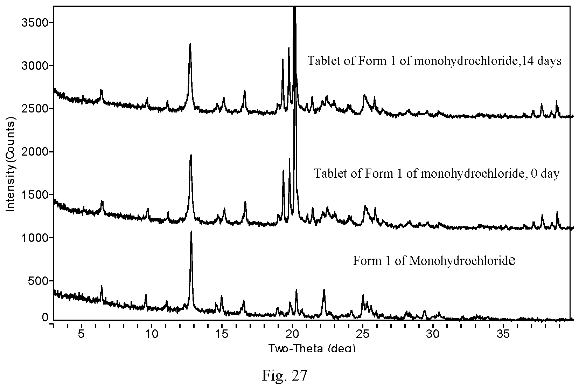

The tablets containg Form 2 of tolebrutinib monohydrochloride Form 2 tablets and Form 1 of tolebrutinib monohydrochloride were placed at 25°C/60% RH for 14 days, respectively. XRPD tests were performed before and after storage. The XRPD comparison plots before and after storage are shown in FIG. 26 and FIG. 27, respectively.

The results showed that the tablets containing Form 2 of tolebrutinib monohydrochloride and tablets ontaining Form 1 of tolebrutinib monohydrochloride remain stable for at least 14 days under the 25°C/60% RH condition.

The above examples are the preferred embodiments of the application, but the implementaion of the application is not limited to the above examples. Any modifications, alterations, substitutions, combinations, simplifications, or changes made within the scope of the spirit and essence of the present application, which do not depart from the scope of the present application, are considered to be equivalent alternatives and are encompassed within the scope of protection of the present application.

## Claims

1. A tolebrutinib monohydrochloride, with a structure represented by Formula (II):

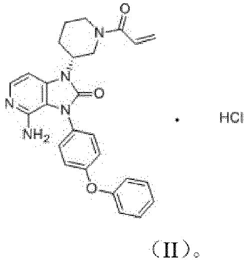

2. The Form 2 of tolebrutinib monohydrochloride according to claim 1, wherein the X-ray powder diffraction pattern comprises at least one characteristic peaks at 2Θ values of 10.2° ± 0.2°, 12.1° ± 0.2°, 15.8° ± 0.2°, 16.2° ± 0.2° and 17.4° ± 0.2°; preferably comprises at least three characteristic peaks; more preferably comprises at least four characteristic peaks.

3. Form 2 of tolebrutinib monohydrochloride according to claim 2, wherein the XRPD pattern of Form 2 comprises at least one characteristic peak at 2Θ values of 15.0°±0.2°, 19.2°±0.2°, 20.3°±0.2°and 21.2°±0.2°2θ.

4. Form 2 of tolebrutinib monohydrochloride Form 2 according to claim 2 or 3, wherein the XRPD pattern of Form 2 comprises one characteristic peak at 2Θ values of 8.1°±0.2°, 13.4°±0.2°, 14.5±0.2°, 23.0°±0.2°, 24.3°±0.2° and 25.1°±0.2°2θ.

5. Form 2 of tolebrutinib monohydrochloride according to any one of claims 2 to 4, wherein the XRPD pattern of Form 2 comprise characteristic peaks at the following 2Θ values:
| 2θ±0.2° |
|---|
| 8.1 |
| 10.2 |
| 12.1 |
| 13.4 |
| 14.5 |
| 15.0 |
| 15.8 |
| 16.2 |
| 16.8 |
| 17.4 |
| 19.2 |
| 20.3 |
| 20.9 |
| 21.2 |
| 21.5 |
| 22.0 |
| 22.5 |
| 22.7 |
| 23.0 |
| 23.4 |
| 23.8 |
| 24.3 |
| 25.1 |
| 29.5 |
| 30.2 |

6. Form 2 of tolebrutinib monohydrochloride according to any one of claims 2 to 5, wherein Form 2 has an XRPD pattern substantially as shown in FIG. 1.

7. Form 2 of tolebrutinib monohydrochloride according to any one of claims 2 to 6, wherein the Fourier transform infrared (FT-IR) spectrum of Form 2 has at least one characteristic peak at1721cm⁻¹±2cm⁻¹, 1682cm⁻¹±2cm⁻¹, 1649cm⁻¹±2cm⁻¹, 1607cm⁻¹±2cm⁻¹, 1509cm⁻¹±2cm⁻¹, 1489cm⁻¹±2cm⁻¹, 1443cm⁻¹±2cm⁻¹, 1240cm⁻¹±2cm⁻¹, 870cm⁻¹±2cm⁻¹, 782cm⁻¹±2cm⁻¹, 757cm⁻¹±2cm⁻¹ and 699cm⁻¹±2cm⁻¹.

8. Form 2 of tolebrutinib monohydrochloride according to any one of claims 2 to 7, wherein Form 2 is anhydrous.

9. A method for preparing the tolebrutinib monohydrochloride according to claim 1, wherein the method comprising:Dissolving tolebrutinib in solvent 1 to form a solution or a supersaturated solution, then mixing it with hydrochloric acid solution to obtain tolebrutinib monohydrochloride;
wherein, the hydrochloric acid solution is a solution of concentrated hydrochloric acid diluted with solvent 2. Solvent 1 and solvent 2 are selected from ethanol, methanol, n-propanol, isopropanol, toluene, ethyl acetate, acetonitrile, or any combination thereof; Preferably, the molar ratio (mol/mol) of tolebrutinib to hydrochloric acid is 1:0.8 to 1:1.2; more preferably 1:1.

10. A method for preparing Form 2 of tolebrutinib monohydrochloride according to any one of claims 2 to 8, wherein the method comprising:Dissolving tolebrutinib in solvent 3 to form a solution or a supersaturated solution, then mixing it with hydrochloric acid solution, stirring, separating, and drying to obtain tolebrutinib monohydrochloride Form 2;
wherein, the hydrochloric acid solution is a solution of concentrated hydrochloric acid diluted with solvent 4. Solvent 3 and solvent 4 are selected from ethanol, methanol, n-propanol, isopropanol, and toluene, or any combination thereof;
preferably, both solvent 3 and solvent 4 are selected from ethanol;
preferably, the mass/volume ratio (mg/mL) of concentrated hydrochloric acid to solvent 4 is 100:1 to 2:1, further preferably 50:1 to 5:1, most preferably 30:1 to 10:1; the concentration of hydrochloric acid in the concentrated hydrochloric acid is 37% (w/w);
preferably, the molar ratio of tolebrutinib to hydrochloric acid is 1:0.8-1:1.2;
preferably, the stirring is performed at room temperature;
preferably, the stirring time is greater than or equal to 2 days, further preferably 3 to 20 days.

11. Form 1 of tolebrutinib monohydrochloride according to any one of claim 1, wherein the X-ray powder diffraction (XRPD) pattern comprises at least four characteristic peaks at 20 values of 6.4°±0.2°, 12.8°±0.2°, 19.0°±0.2°, 20.3°±0.2°, and 22.3°±0.2°.

12. Form 1 of tolebrutinib monohydrochloride according to claim 11, wherein the XRPD pattern of Form 1 comprises one or two or more characteristic peaks at 20 values of 9.6°±0.2°, 15.0°±0.2°, 24.2°±0.2°, 25.0±0.2°and 25.6°±0.2°.

13. Form 1 of tolebrutinib monohydrochloride according to claim 11 or 12, wherein the XRPD pattern of Form 1 also comprises one or two or more characteristic peaks at 20 values of 11.1°±0.2°, 14.6°±0.2°, 16.5°±0.2°and19.8°±0.2°.

14. Form 1 of tolebrutinib monohydrochloride according to any of claims 11 to 13, wherein the XRPD pattern of Form 1 comprise characteristic peaks at the following 20 values:
| 2θ±0.2° |
|---|
| 6.4 |
| 9.6 |
| 11.1 |
| 12.4 |
| 12.8 |
| 14.6 |
| 15.0 |
| 16.5 |
| 19.0 |
| 19.8 |
| 20.3 |
| 22.3 |
| 22.7 |
| 24.2 |
| 25.0 |
| 25.3 |
| 25.6 |
| 28.1 |

15. Form 1 of tolebrutinib monohydrochloride according to any of claims 11 to 14, wherein Form 1 has an XRPD pattern substantially as shown in FIG. 8.

16. Form 1 of tolebrutinib monohydrochloride according to any of claims 11 to 15, wherein the Fourier transform infrared (FT-IR) spectrum of Form 1 has characteristic peaks at 1722±2 cm⁻¹, 1682±2 cm⁻¹, 1650±2 cm⁻¹, and 1607±2 cm⁻¹.

17. Form 1 of tolebrutinib monohydrochloride according to any of claims 11 to 16, wherein the Fourier transform infrared (FT-IR) spectrum of Form 1 has one or two or more characteristic peaks at 1509±2cm⁻¹, 1489±2 cm⁻¹, 1444±2 cm⁻¹, 1240±2 cm⁻¹, 1164±2 cm⁻¹, 1137±2 cm⁻¹, 782±2 cm⁻¹ and 699±2 cm⁻¹.

18. Form 1 of tolebrutinib monohydrochloride according to any of claims 11 to 17, wherein Form 1 is anhydrous.

19. A method for preparing Form 1 of tolebrutinib monohydrochloride according to any of claims 11 to 17, wherein the method is selected from the following methods:
1) dissolving tolebrutinib in solvent 5 to form a solution or a supersaturated solution, then mixing it with hydrochloric acid solution, stirring, separating, and drying to obtain Form 1 of tolebrutinib monohydrochloride;
wherein, the hydrochloric acid solution is a solution of concentrated hydrochloric acid diluted with solvent 6; Solvent 5 and solvent 6 are selected from any one of ethyl acetate, acetonitrile, a mixture of acetonitrile and water, or a mixture of ethyl acetate, acetonitrile, and water;
preferably, both solvent 5 and solvent 6 are ethyl acetate;
preferably, the mass/volume ratio (mg/mL) of concentrated hydrochloric acid to ethyl acetate is 100:1 to 2:1, further preferably 50:1 to 5:1, most preferably 30:1 to 10:1; the concentration of hydrochloric acid in the concentrated hydrochloric acid is 37% (w/w);
preferably, the molar ratio of tolebrutinib to hydrochloric acid is 1:0.8 to 1:1.2;
preferably, the stirring is performed at room temperature.
2) dissolving tolebrutinib in solvent 7, then adding crystal seeds of Form 1 of tolebrutinib monohydrochloride, then mixing it with hydrochloric acid solution, stirring, separating, and drying to obtain Form 1 of tolebrutinib monohydrochloride;
wherein, the hydrochloric acid solution is a solution of concentrated hydrochloric acid diluted with solvent 8; Solvent 7 and solvent 8 are selected from toluene, ethanol, methanol, n-propanol, isopropanol, or any combination thereof;
preferably, the mass ratio (mg/mg) of the tolebrutinib to the seeds of Form 1 of tolebrutinib monohydrochloride is less equal to or less than 10:1, further preferably in the range of 10:5 to 10:3;
preferably, the mass/volume ratio (mg/mL) of concentrated hydrochloric acid to solvent 4 is 100:1 to 2:1, further preferably 50:1 to 5:1, more preferably 30:1 to 10:1; the concentration of hydrochloric acid in the concentrated hydrochloric acid is 37% (w/w);
preferably, the molar ratio of tolebrutinib to hydrochloric acid is 1:0.8 to 1:1.2;
preferably, the stirring is performed at room temperature.

20. A tolebrutinib maleate, with a chemical structure represented by Formula (III): 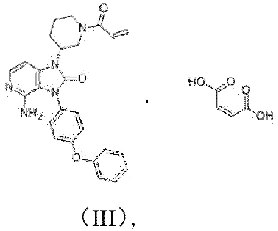 wherein the maleate is a monomaleate.

21. Form 1 of tolebrutinib maleate according to claim 20, wherein the X-ray powder diffraction (XRPD) pattern of maleate Form 1comprises at least four characteristic peaks at 20 values of 7.0°±0.2°, 13.4°±0.2°, 16.3°±0.2°, 17.6°±0.2°, and 20.0°±0.2°.

22. Form 1 of tolebrutinib maleate according to claim 21, wherein the XRPD pattern of maleate Form 1 comprises one or two or more characteristic peaks at 20 values of 16.9±0.2°, 19.1°±0.2°, 21.9±0.2°, 25.0°±0.2°, and 25.9°±0.2°.

23. Form 1 of tolebrutinib maleate according to claim 21 or 22, wherein the XRPD pattern of maleate Form 1 comprises one or two or more characteristic peaks at 20 values of 6.6°±0.2°, 10.3°±0.2°, 21.1°±0.2° and 23.0±0.2°.

24. Form 1 of tolebrutinib maleate according to any one of claims 21 to 23, wherein the XRPD pattern of tolebrutinib maleate Form 1 comprise characteristic peaks at the following 20 values:
| 2θ±0.2° |
|---|
| 6.6 |
| 7.0 |
| 10.3 |
| 12.9 |
| 13.4 |
| 16.3 |
| 16.9 |
| 17.6 |
| 19.1 |
| 20.0 |
| 21.1 |
| 21.9 |
| 23.0 |
| 25.0 |
| 25.9 |

25. Form 1 of tolebrutinib maleate according to claims any one of 21 to 24, wherein maleate Form 1 has an XRPD pattern substantially as shown in FIG. 17.

26. Form 1 of tolebrutinib maleate according to claims any one of 21 to 25, wherein the Fourier transform infrared (FT-IR) spectrum of maleate Form 1 of comprises characteristic peaks at 1734±2cm⁻¹, 1561±2cm⁻¹, 1357±2cm⁻¹, and 1195±2cm⁻¹.

27. Form 1 of tolebrutinib maleate according to any one of claims 21 to 26, wherein the Fourier transform infrared (FT-IR) spectrum of tolebrutinib maleate Form 1 also comprises one or two or more characteristic peaks at 1699±2cm⁻¹, 1630±2cm⁻¹, 1509±2cm⁻¹, 1486±2cm⁻¹, 1396±2cm⁻¹, 1230±2cm⁻¹, 869±2cm⁻¹ and 787±2cm⁻¹.

28. Form 1 of tolebrutinib maleate according to any one of claims 21 to 27, wherein tolebrutinib maleate Form 1 is anhydrous.

29. A method for preparing Form 1 of tolebrutinib maleate according to any one of claims 21 to 28, wherein the method comprising:
dissolving tolebrutinib in solvent 9, then mixing it with maleic acid, stirring, separating, and drying to obtain tolebrutinib maleate Form 1;
preferably, the molar ratio (mol/mol) of tolebrutinib to maleic acid is 1:0.8 to 1:1.2;
preferably, solvent 9 is selected from ethanol, ethyl acetate, water, tetrahydrofuran, 1,4-dioxane, ether, methanol, trifluoroethanol, n-propanol, isopropanol, n-butanol, propylene glycol, and acetone, or any combination thereof; more preferably, ethanol or ethyl acetate;
preferably, the stirring is performed at room temperature.

30. A pharmaceutical composition, comprising one or more of Form 2 of tolebrutinib monohydrochloride according to any one of claims 2 to 8, Form 1 of tolebrutinib monohydrochloride according to any one of claims 11 to 18, Form 1 of tolebrutinib maleate according to any one of claims 21 to 28, and at least one pharmaceutically acceptable carrier.

31. A use of one or more of Form 2 of tolebrutinib monohydrochloride according to any one of claims 2 to 8, Form 1 of tolebrutinib monohydrochloride according to any one of claims 11 to 18, Form 1 of tolebrutinib maleate according to any one of claims 21 to 28, or the pharmaceutical composition thereof in the preparation of a drug for the treatment of BTK-mediated diseases.

32. A use of one or more of Form 2 of tolebrutinib monohydrochloride according to any one of claims 2 to 8, Form 1 of tolebrutinib monohydrochloride according to any one of claims 11 to 18, Form 1 of tolebrutinib maleate according to any one of claims 21 to 28, or the pharmaceutical composition thereof in the preparation of a drug for the treatment of cancers, autoimmune diseases, inflammatory diseases, and thrombotic diseases.

33. A method for treating BTK-mediated diseases, comprising administering an effective amount of one or more of Form 2 of tolebrutinib monohydrochloride according to any one of claims 2 to 8, Form 1 of tolebrutinib monohydrochloride according to any one of claims 11 to 18, Form 1 of tolebrutinib maleate according to any one of claims 21 to 28, or the pharmaceutical composition thereof to a patient.

34. A method for treating cancers, autoimmune diseases, inflammatory diseases, and thrombotic diseases, comprising administering to a patient in need thereof a therapeutically effective amount of one or more Form 2 of tolebrutinib monohydrochloride according to any one of claims 2 to 8, Form 1 of tolebrutinib monohydrochloride according to any one of claims 11 to 18,Form 1 of tolebrutinib maleate according to any one of claims 21 to 28, or the pharmaceutical composition thereof to a patient.

35. A combination use of one or more of Form 2 of tolebrutinib monohydrochloride according to any one of claims 2 to 8, Form 1 of tolebrutinib monohydrochloride according to any one of claims 11 to 18, Form 1 of tolebrutinib maleate according to any one of claims 21 to 28, or the pharmaceutical composition thereof, with other drugs.
